# EUROPÄISCHE PATENTSCHRIFT

(11) **EP 0 710 303 B1**
(45) Veröffentlichungstag und Bekanntmachung des Hinweises auf die Patenterteilung: **26.02.1997**
(21) Anmeldenummer: 94928870.8
(22) Anmeldetag: 06.10.1994
(51) Int. Cl.: D03D 23/00

(54) **SAUGFÄHIGES GEWEBEMATERIAL AUS SYNTHETISCHER ENDLOSFASER**
ABSORBENT FABRIC MADE FROM CONTINUOUS SYNTHETIC FIBRE
TISSU ABSORBANT EN FILS SYNTHETIQUES CONTINUS

(30) Priorität: 19.10.1993 DE 4335621
(43) Veröffentlichungstag der Anmeldung: 08.05.1996
(73) Patentinhaber: ROTECNO AG, CH-8045 Zürich (CH)
(72) Erfinder: ELSENER, Hugo, CH-6855 Stabio (CH)
(74) Vertreter: Morgan, James Garnet
(86) Internationale Anmeldenummer: EP9403309
(87) Internationale Veröffentlichungsnummer: WO9511328

(56) Entgegenhaltungen:
- EP-A- 0 269 207
- EP-A- 0 517 687
- US-A- 4 281 688

## Beschreibung

Die Erfindung betrifft ein saugfähiges Gewebematerial aus synthetischer Endlosfaser, insbesondere zur Verwendung im Klinik- und Reinstraumbereich sowie in betrieblichen und öffentlichen Waschräumen. Ein derartiges Gewebe ist aus der Druckschrift EP-A-0 269 207 bekannt.

Im Klinikbereich, insbesondere im Operationssaal, ebenso wie in Reinsträumen bringt die Verwendung von Gewebematerial aus natürlichen Fasern Probleme mit sich, da natürliche Fasern wegen ihres Aufbaus und ihrer Struktur Fussel und Flusen bilden, die Träger von Keimen und anderen Verschmutzungen sind und sich in der Raumluft verteilen. Ein weiterer Nachteil besteht darin, daß durch die lange Trocknungszeit gequollener Naturfasern, wie zum Beispiel Baumwolle, ein günstiger Nährboden für das Wachsen von Bakterien und Keimen erzeugt wird.

Um den Reinstraumbedingungen bzw. den Hygieneanforderungen zu genügen, werden daher sowohl im Klinikbereich als auch im Reinstraumbereich in zunehmendem Maße natürliche Faserstoffe vermieden und statt dessen so weit wie möglich synthetische Faserstoffe verwendet. Da synthetische Fasern als Endlosgarn herstellbar sind und eine hohe Bruchfestigkeit aufweisen, tritt bei synthetischem Gewebematerial keine Fussel-oder Flusenbildung auf. Nachteilig ist bei synthetischem Gewebematerial jedoch, daß dieses nicht oder nur sehr wenig saugfähig ist. Dieser Nachteil kann im Klinikbereich bei Abdecktüchern und Kleidungsstücken in Kauf genommen werden, bei Handtüchern und Tüchern zum Aufsaugen von Flüssigkeiten ist dies jedoch nicht möglich. Es werden daher ungeachtet der Nachteile von Gewebematerial aus natürlichen Fasern im Klinikbereich Handtücher und Saugtücher insbesondere aus Baumwolle verwendet.

Ein weiterer Nachteil von natürlichen Gewebematerialien besteht darin, daß diese zur Entfernung von Schmutz und zur Abtötung von Keimen bei hoher Temperatur, insbesondere 95°C, und mit einer hohen Waschmittelmenge gewaschen werden müssen, während synthetische Materialien bereits bei einer Waschtemperatur von 60°C hygienisch sauber werden und eine geringere Waschmittelmenge erforderlich ist. Zudem ist die zur Trocknung aufzuwendende Energie bei synthetischen Materialien geringer als bei natürlichen Gewebematerialien, da natürliche Gewebematerialien Feuchtigkeit aufnehmen und dadurch aufquellen, während bei synthetischen Materialien die Feuchtigkeit nur äußerlich anhaftet.

Bei synthetischen Materialien in Tuchform besteht neben der geringen Saugfähigkeit der Nachteil, daß diese sich zu glatt und plastikartig anfühlen und daher vom Griff her eine geringe Akzeptanz für Stoffe aus diesen Materialien besteht, wenn sie als Handtücher, von denen Saugfähigkeit gefordert wird, verwendet werden sollen. Ein weiterer Nachteil besteht in dem sogenannte Pilling-Effekt. Hiermit wird eine Knötchenbildung bezeichnet, die bei synthetischen Kurzfasern auftritt, welche im Gegensatz zu natürlichen Fasern wie Baumwollfasern bei mechanischer Beanspruchung nicht brechen, sondern bei Wechselbeanspruchung Knötchen bilden.

Ziel der vorliegenden Erfindung ist es daher, ein saugfähiges Material aus synthetischer Faser zur Verfügung zu stellen, das sich durch besondere Saugfähigkeit und ausreichende Griffakzeptanz auszeichnet.

Ein derartiges Material ist in Patentanspruch 1 angegeben. Durch die Verwendung einer bauschfähigen Faser, welche in einer offenen Webstruktur zu einem Gewebe verwoben ist, entstehen längere nicht abgebundene Fadenabschnitte, die sich mit fest abgebundenen Fadenabschnitten abwechseln. Diese zwischen fest abgebundenen Fadenabschnitten vorhandenen, nicht abgebundenen Fadenabschnitte ermöglichen ein Aufbauschen der bauschfähigen Faser. Bei mechanischer Beanspruchung des Gewebematerials, beispielsweise beim Knüllen des Materials zum Händetrocknen, werden diese Fadenabschnitte abwechselnd gedehnt und gestaucht, wodurch ein Pumpeffekt entsteht, aufgrund dessen Flüssigkeit in die kapillarartigen Faserzwischenräume des Gewebematerials gesogen wird. Die aufgesogene Flüssigkeit wird von dem Gewebematerial insbesondere durch Adhäsionskräfte zurückgehalten und dadurch gespeichert. Wichtig für das Entstehen der Pumpwirkung ist somit, daß eine Vielzahl von zwischen zwei fest eingebundenen, gequetschten Stellen vorhandene freie, nicht gequetschte Fadenabschnitte vorhanden sind, in denen sich die Faser aufbauschen kann.

Durch das Aufbauschen erhöht sich auch die Oberfläche der im Gewebe vorhandenen Faser und dadurch die Aufnahmefähigkeit des Gewebes für Flüssigkeit. Die Flüssigkeit wird also nicht wie bei Naturfasern von der Faser durch Quellung aufgenommen, sondern von dem Gewebe, indem sie in die kapillarartigen Faserzwischenräume des Gewebematerials gesaugt und durch Adhäsionskräfte verteilt und gebunden wird.

Im Gebrauch wirkt sich vom hygienischen Standpunkt positiv aus, daß durch das rasche Trocknen von Synthetikgeweben - das Wasser ist nur in den Faserzwischenräumen und nicht in der gequollenen Faser gebunden - das Wachsen von Bakterien und Keimen reduziert wird.

Durch die Verwendung von synthetischen Endlos-Fasern wird bei dem erfindungsgemäßen Gewebematerial trotz hoher Saugfähigkeit die Bildung von Fusseln oder Flusen vermieden, so daß aus diesem Material hergestellte Gegenstände, insbesondere Handtücher, problemlos im Klinik- und Reinstraumbereich eingesetzt werden können. Es treten daher auch keine Probleme auf, wenn Handtücher aus diesem Material gemeinsam mit anderen synthetischen Tüchern wie Abdecktüchern oder Kleidungsstücken gemeinsam gewaschen werden. Werden nämlich Baumwollhandtücher gemeinsam mit diesen anderen Gegenständen gewaschen, so haften die von den Baumwollhandtüchern stammenden Flusen und Fussel auch an den übrigen Gegenständen und müssen von diesen mühsam und im Regelfall von Hand entfernt werden.

Ein weiterer Vorteil besteht darin, daß Stoffe aus dem erfindungsgemäßen Material eine geringere Waschtemperatur und eine geringere Waschmittelmenge benötigen sowie eine geringere Trocknungsenergie. Stoffe aus dem erfindungsgemäßen Material können daher besonders wirtschaftlich verwendet werden.

Nach einer Ausgestaltung der Erfindung sind etwa 60 % bis 80 %, bevorzugt etwa 70 %, der Fadenkreuzstellen nicht abgebunden. Ein derartiges Gewebe verbindet eine hohe Saugfähigkeit mit einer guten Griffakzeptanz. Dies ergibt sich daraus, daß bei dem angegebenen Verhältnis von abgebundenen zu nicht abgebundenen Fadenkreuzstellen einerseits ausreichend freie Fadenabschnitte vorhanden sind, die die Saugfähigkeit erhöhen, und andererseits ausreichend viele abgebundene Fadenkreuzstellen vorhanden sind, um eine gute Schiebefestigkeit zu erreichen und die Gewebeoberfläche stark zu strukturieren.

Nach einer weiteren Ausgestaltung der Erfindung sind die abgebundenen Fadenkreuzstellen thermisch, insbesondere während des Färbevorgangs, fixiert. Hierdurch wird vorteilhafterweise die optimale Gewebestruktur fixiert und dadurch die Saugfestigkeit und Griffakzeptanz dauerhaft gewährleistet.

Nach einer weiteren Ausgestaltung der Erfindung ist das Gewebe aus einer Multifilamentfaser gebildet. Eine solche Faser hat eine besonders große Oberfläche, und die Faserzwischenräume sind besonders fein, wodurch die Saugfähigkeit des daraus gebildeten Gewebematerials erhöht wird. Eine Multifilamentfaser kann darüber hinaus besonders bauschfähig ausgebildet werden, wodurch der Pumpeffekt des Gewebes erhöht wird.

Das Aufbauschen der Multifilamentfaser erfolgt nach einer weiteren Ausgestaltung der Erfindung bevorzugt durch Kräuseln, also durch Verdrehung der Einzelfilamente unter Temperatureinfluß während der Herstellung des Multifilaments. Bevorzugt wird für die Herstellung des Gewebes ein hochbauschfähiges Material wie falschdrahtextrudiertes oder friktionsextrudiertes Polyestergarn verwendet.

Nach einer weiteren Ausgestaltung der Erfindung weisen die Einzelfilamente eine Dicke von 0,5 dtex bis 5 dtex, insbesondere von 1 dtex bis 2,5 dtex, also Gramm pro 10.000 m auf. Durch diese Ausgestaltung weist das Gewebe einerseits eine sehr große Oberfläche auf und andererseits eine hohe Anzahl von kapillarartigen Räumen zwischen den Fasern auf. Das von dem Multifilament gebildete Garn weist dabei bevorzugt eine Stärke von 50 dtex bis 500 dtex auf, wobei in einem Gewebe auch Fasern verschiedener Stärke verwendet werden können.

Nach einer weiteren Ausgestaltung der Erfindung sind die Fasern auf chemischem Wege hydrophil ausgerüstet. Hierdurch wird vorteilhafterweise die Saugfähigkeit und die Speicherfähigkeit des Gewebes erhöht.

Nach einer weiteren Ausgestaltung der Erfindung sind die Fasern bzw. das von den Fasern gebildete Gewebe antistatisch ausgerüstet. Ein derart behandeltes Gewebe hat den Vorteil, daß es nicht nur selbst flusen- und fusselfrei ist, sondern auch keine fremden Flusen oder Fussel aufgrund elektrostatischer Aufladung anzieht.

Nach einer weiteren Ausgestaltung der Erfindung weist das Gewebe leitfähige Fasern auf, insbesondere Fasern gleicher mechanischer Eigenschaften wie die nicht leitfähigen Fasern. Hierdurch wird vorteilhafterweise eine elektrostatische Aufladung des Gewebematerials vermieden. Es können alternativ auch einzelne Filamente jeder Faser leitfähig ausgebildet sein.

Nach einer weiteren Ausgestaltung der Erfindung weisen die Filamente der Fasern des erfindungsgemäßen Gewebes neben rundem und ovalförmigem auch drei-, vier- oder mehreckigen Querschnitt auf. Durch diese Ausgestaltung kann die Saugfähigkeit des erfindungsgemäßen Gewebematerials weiter erhöht werden, da hierdurch eine Oberflächenstruktur erzeugt wird, die die Flüssigkeitsaufnahme fördert.

Eine solche Förderung der Aufnahmefähigkeit kann nach einer weiteren Ausgestaltung der Erfindung auch dadurch bewirkt werden, daß die Oberfläche der Faserfilamente des erfindungsgemäßen Gewebes selbst strukturiert ist. Auf dem Gewebe insbesondere in Tropfenform haftende Flüssigkeit wird durch diese Ausgestaltung der Fasern leichter von dem Gewebe aufgesogen, da die Rauhigkeit der Oberfläche die Oberflächenspannung reduziert und dadurch eine Verteilung des Tropfens fördert.

Eine bevorzugte Verwendungsweise des erfindungsgemäßen Gewebematerials stellen Handtücher, insbesondere im Klinik- und Reinstraumbereich, sowie Rollenhandtücher im öffentlichen und gewerblichen Bereich dar. Rollenhandtücher im gewerblichen Bereich, beispielsweise in Gaststätten, werden sehr häufig gewaschen, so daß sich die wirtschaftlichen Vorteile hierbei besonders bemerkbar machen.

Ein Ausführungsbeispiel der Erfindung ist in der Zeichnung dargestellt und wird nachfolgend beschrieben.

Es zeigen
- Figur 1: eine erfindungsgemäße Gewebestruktur in vergrößerter schematischer Darstellung und
- Figur 2: einen Schnitt durch ein erfindungsgemäßes Gewebe entsprechend Linie II-II in Figur 1 in stark vereinfachter Darstellung.

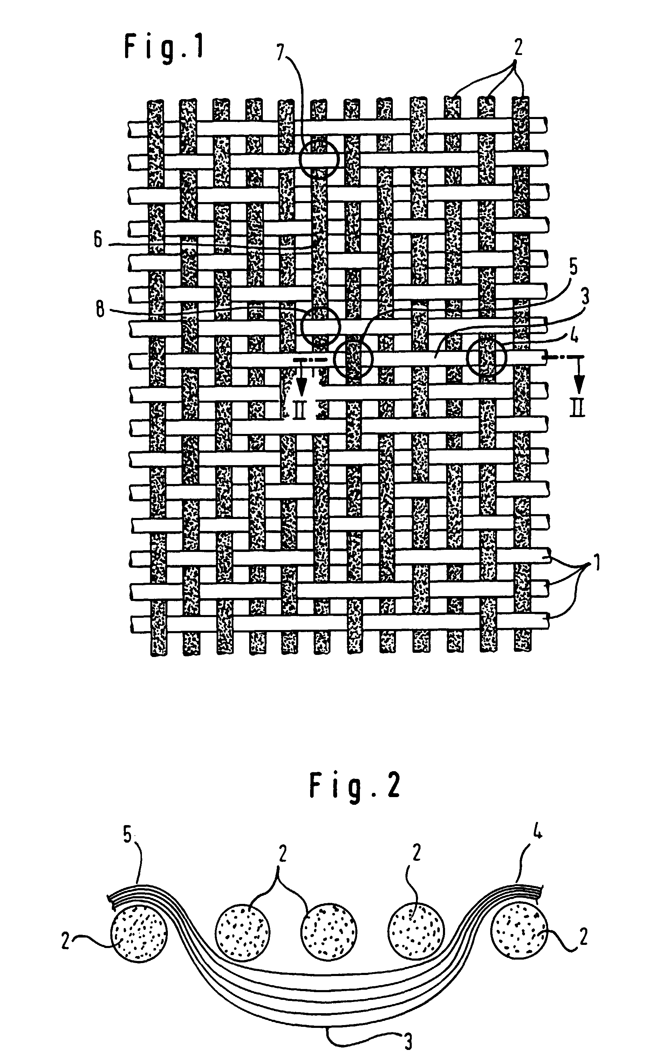

Bei der in Figur 1 dargestellten Gewebestruktur sind die Kettfäden 1 weiß und die hierzu senkrecht verlaufenden Schußfäden 2 schwarz dargestellt. Die dargestellte Struktur zeigt eine Vielzahl von nicht abgebundenen Fadenabschnitten wie beispielsweise den Kettfadenabschnitt 3, die zwischen fest abgebundenen Fadenabschnitten wie die Kettfadenabschnitte 4 und 5 eingebunden sind. Solche nicht eingebundenen Fadenabschnitte sind auch bei den Schußfäden 2 vorhanden, beispielsweise der Fadenabschnitt 6 zwischen den fest eingebundenen Bereichen 7 und 8. Durch diese Ausgestaltung wird eine hohe Saugfähigkeit des Gewebes verbunden mit einer starken Strukturierung der Gewebeoberfläche erreicht.

Der in Figur 2 dargestellte Schnitt durch ein erfindungsgemäßes Gewebe zeigt in einer stark vereinfachten Seitenansicht einen nicht abgebundenen Kettfadenabschnitt 3, der sich zwischen zwei fest abgebundenen Kettfadenabschnitten 4 und 5 befindet. In diesem nicht abgebundenen Fadenabschnitt 3 ist der Kettfaden 1 aufgebauscht, während er in den fest abgebundenen Abschnitten 4 und 5 gequetscht ist. Durch eine mechanische Beanspruchung des Gewebes werden sowohl die Kettfäden 1 als auch die Schußfäden 2 gegeneinander bewegt. Der nicht gebundene Bereich 3 des Kettfadens 1 wird dadurch abwechselnd stärker und schwächer aufgebauscht, wodurch ein Pumpeffekt entsteht, der Flüssigkeit in die Zwischenräume zwischen den Einzelfilamenten des Kettfadens 1 saugt. In diesen Zwischenräumen wird die Flüssigkeit durch Kapillar- und Adhäsionskräfte festgehalten und dadurch in dem Gewebe gespeichert. Dasselbe tritt in den nicht abgebundenen Bereichen der Schußfäden auf, so daß insgesamt eine große Menge Flüssigkeit aufsaugbar und speicherbar ist.

Die Kett- und Schußfäden können eine Stärke insbesondere zwischen 50 dtex bis 500 dtex aufweisen, wobei beispielsweise die Kette 76 dtex haben kann und der Schuß 167 dtex. Die Einzelfilamente können bevorzugt eine Stärke zwischen 0,5 dtex bis 5 dtex aufweisen, insbesondere können Einzelfilamente im Einzeltiterbereich von 1 dtex bis 2,5 dtex verwendet werden. Die Anzahl der Filamente in einem Faden kann beispielsweise 128 betragen.

Ein in der angegebenen Weise hergestelltes Gewebematerial ist besonders für die Herstellung von Handtüchern geeignet, da dessen Griff dem von Handtüchern aus natürlichem Gewebe entspricht. Insbesondere kann es sich um Handtücher handeln, wie sie im Klinikbereich und in Rollhandtuchgeräten verwendet werden.

Durch ein Kräuseln der Multifilamentfasern entsteht ein wirrer Verbund und dadurch ein Multifilamentgarn mit einer Vielzahl von Innenhohlräumen, die von außen zugänglich sind und der Aufnahme von Flüssigkeit dienen können. Durch die Kräuselung entsteht beispielsweise aus einem ursprünglich 10.000 m langen Multifilament ein gekräuseltes Garn mit einer Länge von 9.000 m.

## Patentansprüche

1. Saugfähiges Gewebematerial aus synthetischer Endlosfaser, insbesondere zur Verwendung im Klinik- und Reinstraumbereich sowie in betrieblichen und öffentlichen Waschräumen,
dadurch **gekennzeichnet**,
daß die Faser (1, 2) des Gewebematerials bauschfähig ist und daß das gewobene Material eine offene Webstruktur aufweist, bei welcher sich längere nicht abgebundene Fadenabschnitte (3, 6) mit fest abgebundenen Fadenabschnitten (4, 5 bzw. 7, 8) abwechseln.

2. Gewebematerial nach Anspruch 1,
dadurch **gekennzeichnet**,
daß die Webstruktur etwa 60 % bis 80 %, bevorzugt 70 %, nicht abgebundene Fadenkreuzstellen aufweist.

3. Gewebematerial nach Anspruch 1 oder 2,
dadurch **gekennzeichnet**,
daß die Fadenkreuzstellen thermisch fixiert sind.

4. Gewebematerial nach einem der Ansprüche 1 bis 3,
dadurch **gekennzeichnet**,
daß die synthetische Endlosfaser aus einer Vielzahl von Einzelfasern zusammengesetzt ist.

5. Gewebematerial nach Anspruch 4,
dadurch **gekennzeichnet**,
daß die Einzelfilamente der Faser gekräuselt sind.

6. Gewebematerial nach Anspruch 5,
dadurch **gekennzeichnet**,
daß als synthetische Endlosfaser eine Hochbauschfaser wie falschdrahtextrudiertes oder friktionsextrudiertes Polyestergarn verwendet wird.

7. Gewebematerial nach einem der Ansprüche 4 bis 6,
dadurch **gekennzeichnet**,
daß die Einzelfilamente eine Stärke von 0,5 dtex bis 5 dtex, insbesondere von 1 dtex bis 2,5 dtex, also Gramm pro 10.000 m, aufweisen.

8. Gewebematerial nach einem der vorhergehenden Ansprüche,
dadurch **gekennzeichnet**,
daß die synthetische Endlosfaser eine Gesamtstärke von 50 dtex bis 500 dtex aufweist.

9. Gewebematerial nach einem der Ansprüche 4 bis 8,
dadurch **gekennzeichnet**,
daß synthetische Endlosfasern verschiedener Stärke in einem Gewebe verwendet werden.

10. Gewebematerial nach einem der vorhergehenden Ansprüche,
dadurch **gekennzeichnet**,
daß die synthetische Endlosfaser, insbesondere durch chemische Behandlung, hydrophil ausgebildet ist.

11. Gewebematerial nach einem der vorhergehenden Ansprüche,
dadurch **gekennzeichnet**,
daß die synthetische Endlosfaser antistatisch ausgestaltet ist.

12. Gewebematerial nach einem der vorhergehenden Ansprüche,
dadurch **gekennzeichnet**,
daß das Gewebematerial leitfähige Fasern, bevorzugt gleicher mechanischer Eigenschaften wie die übrigen Fasern, enthält.

13. Gewebematerial nach Anspruch 12,
dadurch **gekennzeichnet**,
daß alle oder ein Teil der Fasern leitfähige Filamente enthält.

14. Gewebematerial nach Anspruch 12 oder 13,
dadurch **gekennzeichnet**,
daß das Gewebe Karbonfasern und / oder Karbonfilamente enthält.

15. Gewebematerial nach einem der vorhergehenden Ansprüche,
dadurch **gekennzeichnet**,
daß die Fasern bzw. die Einzelfilamente neben rundem und ovalförmigem auch drei-, vier- oder mehreckigen Querschnitt aufweisen.

16. Gewebematerial nach einem der vorhergehenden Ansprüche,
dadurch **gekennzeichnet**,
daß die Fasern bzw. die Einzelfilamente eine strukturierte Oberfläche aufweisen.

17. Handtuch aus einem Gewebematerial nach einem der vorhergehenden Ansprüche.

## Claims

1. Absorbent fabric material of synthetic endless fibers, in particular for use in clinical areas and in clean room areas and also in company and public washrooms,
characterized in that
the fiber (1, 2) of the fabric material can be bulked and in that the woven material has an open woven structure in which longer non-bound thread sections (3, 6) alternate with firmly bound-in thread sections (4, 5 and 7, 8 respectively).

2. Fabric material in accordance with claim 1,
characterized in that
the woven structure has approximately 60 % to 80 %, preferably 70 % not bound-in thread crossing points.

3. Fabric material in accordance with claim 1 or claim 2,
characterized in that
the thread crossing points are thermally fixed.

4. Fabric material in accordance with one of the claims 1 to 3,
characterized in that
the synthetic endless fiber is put together from a plurality of individual fibers.

5. Fabric material in accordance with claim 4,
characterized in that
the individual filaments of the fibers are crimped.

6. Fabric material in accordance with claim 5,
characterized in that
a highly bulkable fiber such as false twist extruded or friction extruded polyester yarn is used as the synthetic endless fiber.

7. Fabric material in accordance with one of the claims 4 to 6,
characterized in that
the individual filaments have a thickness from 0.5 dtex to 5 dtex, in particular of 1 dtex to 2.5 dtex, i.e. grams per 10,000 m.

8. Fabric material in accordance with one of the preceding claims,
characterized in that
the synthetic endless fibers have a total thickness of 50 dtex to 500 dtex.

9. Fabric material in accordance with one of the claims 4 to 8,
characterized in that
synthetic endless fibers of different thicknesses are used in one fabric.

10. Fabric material in accordance with one of the preceding claims,
characterized in that
the synthetic endless fiber is made hydrophilic, in particular by chemical treatment.

11. Fabric material in accordance with one of the preceding claims,
characterized in that
the synthetic endless fiber is made antistatic.

12. Fabric material in accordance with one of the preceding claims,
characterized in that
the fabric material contains conductive fibers, preferably of the same mechanical characteristics as the remaining fibers.

13. Fabric material in accordance with claim 12,
characterized in that
all or part of the fibers contain conductive filaments.

14. Fabric material in accordance with claim 12 or claim 13,
characterized in that
the fabric contains carbon fibers and/or carbon filaments.

15. Fabric material in accordance with one of the preceding claims,
characterized in that
the fibers or the individual filaments have triangular, rectangular or polygonal cross-section as well as round or oval shaped cross-sections.

16. Fabric material in accordance with one of the preceding claims,
characterized in that
the fibers or the individual filaments have a structured surface.

17. Handtowel of a fabric material in accordance with one of the preceding claims.

## Revendications

1. Tissu absorbant en fibre synthétique continue, en particulier destiné à l'utilisation dans des espaces cliniques et dans des salles blanches ainsi que dans des salles d'eau d'entreprises et publiques, caractérisé en ce que la fibre (1, 2) du tissu est susceptible de se gonfler, et en ce que le matériau tissé présente une structure de tissu ouverte dans laquelle des tronçons de fils (3, 6) longs non liés alternent avec des tronçons de fils liés (4, 5 et 7, 8) respectivement.

2. Tissu selon la revendication 1, caractérisé en ce que la structure tissée présente 60 % à 80%, de préférence 70 % d'emplacements de croisement de fils non liés.

3. Tissu selon l'une ou l'autre des revendications 1 et 2, caractérisé en ce que les emplacements de croisement de fils sont fixés thermiquement.

4. Tissu selon l'une quelconque des revendications 1 à 3, caractérisé en ce que la fibre synthétique continue est composée d'une multitude de fibres individuelles.

5. Tissu selon la revendication 4, caractérisé en ce que les filaments individuels de fibre sont frisés.

6. Tissu selon la revendication 5, caractérisé en ce qu'on utilise comme fibre synthétique continue une fibre à haut effet de gonflement telle que du fil de polyester extrudé par fausse torsion ou extrudé par friction.

7. Tissu selon l'une quelconque des revendications 4 à 6, caractérisé en ce que les filaments individuels présentent une épaisseur de 0,5 dtex à 5 dtex, en particulier de ldtex à 2,5 dtex, c'est-à-dire grammes par 10.000 m.

8. Tissu selon l'une quelconque des revendications précédentes, caractérisé en ce que la fibre synthétique continue présente une épaisseur totale de 50 dtex à 500 dtex.

9. Tissu selon l'une quelconque des revendications 4 à 8, caractérisé en ce qu'on utilise dans un tissu des fibres synthétiques continues de différentes épaisseurs.

10. Tissu selon l'une quelconque des revendications précédentes, caractérisé en ce que la fibre synthétique continue est rendue hydrophile, en particulier par traitement chimique.

11. Tissu selon l'une quelconque des revendications précédentes, caractérisé en ce que la fibre synthétique continue est rendue antistatique.

12. Tissu selon l'une quelconque des revendications précédentes, caractérisé en ce que le tissu contient des fibres conductrices, qui ont de préférence les mêmes propriétés mécaniques que les autres fibres.

13. Tissu selon la revendication 12, caractérisé en ce que toutes les fibres ou une partie de celles-ci contiennent des filaments conducteurs.

14. Tissu selon l'une ou l'autre des revendications 12 et 13, caractérisé en ce que le tissu contient des fibres de carbone et/ou des filaments de carbone.

15. Tissu selon l'une quelconque des revendications précédentes, caractérisé en ce qu'en plus d'une section ronde ou ovale, les fibres ou les filaments individuels, respectivement, présentent aussi une section triangulaire, quadrangulaire ou polygonale.

16. Tissu selon l'une quelconque des revendications précédentes, caractérisé en ce que les fibres ou les filaments individuels, respectivement, présentent une surface structurée.

17. Serviette en un tissu selon l'une quelconque des revendications précédentes.
